# EUROPEAN PATENT APPLICATION

(11) **EP 1 096 011 A1**
(43) Date of publication of application: **02.05.2001**
(21) Application number: 99203518.8
(22) Date of filing: 26.10.1999
(51) Int. Cl.: C12N 15/52, C12N 15/62, C12N 9/00, C07K 5/06

(54) **Microbiological production method for alpha-l-aspartyl-l-phenylalanine**

(71) Applicant: Holland Sweetener Company V.o.F., 6167 RA Geleen (NL)
(72) Inventor: Doekel, Sascha, 35039 Marburg (DE); Marahiel, Mohamed Abdalla, 35043 Marburg (DE); Quaedflieg, Peter Jan Leonard Mario, 6164 HB Geleen (NL); Sonke, Theodorus, 6143 BK Sittard (NL)

(57) **Abstract**

The present invention relates to a new, microbiological, method for the production of α-L-aspartyl-L-phenylalanine (Asp-Phe) from the substrates L-aspartic acid (L-Asp) and L-phenylalanine (L-Phe) wherein the substrates are contacted, in the presence of ATP, with a non-ribosomal dipeptide synthetase comprising two minimal modules connected by one condensation domain wherein the N- resp. C-terminal modules are recognising L-Asp and L-Phe, respectively, and the latter module is covalently bound at its N-terminal end to the condensation domain, and wherein each of these minimal modules is composed of an adenylation domain and a 4'-phosphopantetheinyl cofactor containing thiolation domain, and that the Asp-Phe formed is recovered. The present invention also relates to novel DNA fragments or combination of DNA fragments encoding a new Asp-Phe dipeptide synthetase, micro-organisms containing such DNA fragments, as well as to the new Asp-Phe dipeptide synthetases itself.

## Description

### FIELD OF THE INVENTION

The present invention relates to a new, microbiological, method for the production of α-L-aspartyl-L-phenylalanine (Asp-Phe) from the substrates L-aspartic acid (L-Asp) and L-phenylalanine (L-Phe). The present invention also relates to novel DNA fragments or combination of DNA fragments encoding a new Asp-Phe dipeptide synthetase, micro-organisms containing such DNA fragments, as well as to the new Asp-Phe dipeptide synthetases itself.

### BACKGROUND OF THE INVENTION

α-L-Aspartyl-L-phenylalanine (hereinafter also referred to as Asp-Phe) is an important dipeptide, inter alia used for the production of α-L-aspartyl-L-phenylalanine methyl ester (hereinafter also referred to as APM). APM is known to be a high intensity artificial sweetener, having a sweetness which is about 200x as potent as the sweetness of sucrose. The β-form of APM, as well as the stereoisomers of APM wherein one or both of the amino acids are in the D-configuration, do not have sweet properties. APM is used for the sweetening of various edible materials.

Various production methods of APM exist; present routes may be divided into chemical and biochemical/microbiological (in particular, enzymatic) routes. In the ways of producing APM by using known peptide synthesis techniques tedious and expensive processes have to be performed in order to achieve selective α-L,L-coupling, involving intensive protecting and deprotecting of α-amino, carboxyl and side chain groups. Fermentative routes, on the other hand, in general are cheap and intrinsically they display enantio- and regioselectivity. Therefore, fermentative routes have been considered to be promising alternatives for the above-mentioned chemical and biochemical synthesis routes. As can be seen from EP-A-0036258, it has so far been deemed unsuited to produce the dipeptide Asp-Phe in a micro-organism as part of the micro-organism's own protein producing processes; theoretically such production might be achieved by inserting in the DNA of a micro-organism the nucleotide base sequences GAC or GAT (being known to be a codon for L-Asp) and TTT or TTC (being known to be a codon for L-Phe), preceded and followed by appropriate processing or termination codons in the correct reading frame, and under appropriate control. It therefore has been attempted in EP-A-0036258 to achieve the synthesis of Asp-Phe indirectly through prior production of protein segments of the formula (Asp-Phe)ₙ, where n is a large number; this has been done by inserting into a cloning vehicle a synthesised DNA-fragment coding for such poly-(Asp-Phe) protein. However, such ribosomal fermentative route is still tedious and economically unattractive. Major drawbacks are lying in the recovery of the Asp-Phe dipeptide from the polypeptide. Similar drawbacks can be attributed to a method, as described by Choi, S.-Y. et al. in J. Microbiol. Biotechnol., **2**, 1992, p.1-6, wherein a polypeptide comprising segments of the tripeptide sequence Asp-Phe-Lys is synthesised. Therefore still need exists for finding a direct fermentative route to Asp-Phe. Direct fermentation of Asp-Phe is hitherto unknown.

### DESCRIPTION OF THE INVENTION

### METHOD FOR THE PRODUCTION OF ASP-PHE:

Surprisingly, inventors now found a new, and promising alternative microbiological method for the production of α-L-aspartyl-L-phenylalanine (Asp-Phe) from the substrates L-aspartic acid (L-Asp) and L-phenylalanine (L-Phe) wherein the substrates are contacted, in the presence of an effective amount of adenosine-triphosphate (ATP), with a non-ribosomal dipeptide synthetase comprising two minimal modules connected by one condensation domain wherein the N-terminal module of these modules is recognising L-aspartic acid and the C-terminal module of these modules is recognising L-phenylalanine and is covalently bound at its N-terminal end to the condensation domain, and wherein each of these minimal modules is composed of an adenylation domain and a 4'-phosphopantetheinyl cofactor containing thiolation domain, and that the α-L-aspartyl-L-phenylalanine (Asp-Phe) formed is recovered.

This new method thus provides a microbiological process for direct fermentation of Asp-Phe, which in a subsequent methylation step may be converted into the intense sweetener aspartame. Production of Asp-Phe by direct fermentation is hitherto unknown, as is non-ribosomal synthesis of this dipeptide. The inventors thus have provided a direct microbiological method for producing the dipeptide Asp-Phe without the need for any protecting and deprotecting steps.

The novel non-ribosomal dipeptide synthetases which, according to the present invention, can be used for the production of Asp-Phe are also indicated hereinafter as Asp-Phe dipeptide synthetases or as Asp-Phe synthetases. It is known (for instance, from P. Zuber et al., in *"Bacillus subtilis* and other Gram-positive bacteria", Sonenshein et al. (Eds.), Am. Soc. Microbiol., Washington, DC, 1993, p.897-916) that micro-organisms can produce bioactive peptides through ribosomal and non-ribosomal mechanisms. The bioactive peptides so far known to be synthesised non-ribosomally, are produced by a number of soil bacteria and fungi. These bioactive peptides can range from 2 to 48 residues, and are structurally diverse. They may show a broad spectrum of biological properties including antimicrobial, antiviral or antitumor activities, or immunosuppressive or enzyme-inhibiting activities. As such, these non-ribosomally synthesised bioactive peptides form a class of peptide secondary metabolites which has found widespread use in medicine, agriculture, and biological research. Already more than 300 different residues so far have been found to be incorporated into these peptide secondary metabolites. However, until now not a single non-ribosomally formed peptide has been identified having (as a part of its peptide sequence) the dipeptide Asp-Phe in it, nor has the dipeptide Asp-Phe itself been identified as a non-ribosomally synthesised product.

According to the present invention Asp-Phe can now be produced non-ribosomally, and novel non-ribosomal Asp-Phe synthetases can be used for the synthesis of Asp-Phe. Hereinafter, in the part of the specification dealing with the DNA-fragments encoding the novel Asp-Phe synthetases, it will be elucidated in more detail how these novel Asp-Phe synthetases can be obtained and have been made available in the context of the present invention. For better understanding of the present invention, first, however, some general background as to non-ribosomal peptide synthesis is presented.

In non-ribosomal synthesis of peptides known so far generally a multiple carrier thiotemplate mechanism is involved (T. Stein et al., J. Biol. Chem. **271**, 1996, p.15428-15435). According to this model, peptide bond formation takes place on multi-enzyme complexes which are named peptide synthetases and which comprise a sequence of amino acid recognising modules. On the peptide synthetases a series of enzymatic reactions take place which ultimately lead to the formation of a peptide by sequential building-in of amino acids, in an order predetermined by the order of modules recognising the cognate amino acids, into the peptide. This series of enzymatic reactions includes, schematically:
1. recognition of the amino acid substrates;
2. activation of said recognised amino acid substrates to their aminoacyl-adenylates (that is, the aminoacyl adenosine-monophosphate; aa-AMP) at the expense of Mg²⁺-ATP (adenylation);
3. binding of the aminoacyl-adenylates in the form of their more stable thioesters to the cysteamine group of the enzyme-bound 4'-phosphopantetheinyl (4'-PP) cofactors (thiolation). The ATP consumed in the adenylation reaction is hereby released in the monophosphate form (AMP) ;
4. depending of the peptide to be synthesised non-ribosomally, the thiol-activated substrates may be modified (e.g. by epimerisation or *N*-methylation);
5. formation of the peptide product by N to C stepwise integration of the thioesterified substrate amino acids (modified, as the case may be) into the growing peptide;
6. releasing the peptide formed non-ribosomally from the template.

Assuming this general scheme also to be correct for the novel non-ribosomal synthesis of Asp-Phe according to the present invention, this means that this synthesis involves the subsequent steps of (i) recognition of L-Asp and L-Phe, (ii) formation of an L-Asp- and an L-Phe-acyladenylate, (iii) binding thereof to the cysteamine group of the 4'-PP cofactor in the respective thiolation domains, (iv) formation of the Asp-Phe dipeptide by transfer of the thioester-activated carboxyl group of L-Asp to the amino group of L-Phe, while the condensation product remains covalently attached to the multi-enzyme complex via the 4'-PP cofactor in the thiolation domain of the Phe-recognising module, and (v) release of the Asp-Phe formed.

According to the present invention the substrates L-Asp and L-Phe are contacted with a non-ribosomal Asp-Phe dipeptide synthetase, in the presence of an effective amount of ATP. An effective amount of ATP as meant herein is an amount of ATP which ensures that the dipeptide formation takes place at a suitable rate. Usually such rate will be at least one turn-over per minute, i.e. a turn-over number (*k*_{*cat*}) of 1 per minute; preferably *k*_{*cat*} is at least 10 per minute. In order to enable an economically attractive process the ATP consumed by the peptide synthesis reaction is preferably regenerated.

The contacting of the substrates L-Asp and L-Phe with the non-ribosomal Asp-Phe dipeptide synthetase may be done in any suitable way; for instance - if the Asp-Phe dipeptide synthetase is present in a micro-organism - L-Asp and L-Phe may be fed into the culture medium containing said micro-organism. Alternatively micro-organisms may be used which are capable of overproducing L-Asp and/or L-Phe (e.g. from glucose), with separately feeding to the micro-organism of the amino acid (L-Asp or L-Phe) which is not produced by the micro-organism. All these methods may be called *in vivo* methods. ATP may be regenerated *in vivo* in the Asp-Phe producing micro-organism.

The contacting of the substrates L-Asp and L-Phe with the non-ribosomal Asp-Phe dipeptide synthetase also may be done by using the synthetase in its isolated form, that is by an *in vitro* method. In such *in vitro* methods ATP-regeneration is to be taken care of separately. This may be done by applying an ATP-regeneration system. ATP-regeneration systems are readily available to the skilled man.

Protein chemical studies and recent progress in cloning and sequencing of genes encoding peptide synthetases of bacterial and fungal origin have made it clear that the known peptide synthetases have a highly conserved and ordered structure composed of so-called modules. These modules have been defined as semi-autonomous units within peptide synthetases that carry all information needed for recognition, activation, and modification of one substrate. Although the modules in principle can act independently, it is generally assumed that they have to work in concert, in a template-based mode of action to achieve peptide elongation.

In general, the modules of peptide synthetases, each module being about 1000-1400 amino acids in length (i.e., the modules have molecular weights in the range of 120-160 kDa), are themselves composed as a linear arrangement of conserved domains specifically representing the enzyme activities involved in substrate recognition, activation, (and, optionally, as the case may be, modification) and condensation (i.e. peptide bond formation). Two of such distinct domains, the adenylation and thiolation domains (A-domain and T-domain), together form the smallest part of a module that retains all catalytic activities for specific activation and covalent binding of the amino acid substrate. Stachelhaus et al. have designated this core fragment of the modules as a "minimal module" (T. Stachelhaus et al., J. Biol. Chem. **270**, 1995, p.6163-6169).

The term "minimal module" as used here therefore, according to said definition, refers to such combined core fragment of the modules, consisting of an adenylation domain and a thiolation domain.

Some highly conserved core motifs of adenylation and thiolation domains, as known to exist in peptide synthetases, are listed in table 1, together with some highly conserved core motifs of condensation and thioesterase domains (which will be addressed in more detail in later parts of this specification).

The so-called "adenylation domain" (A-domain, about 550 amino acids) is an essential region of each module. The A-domain has been shown to bear the substrate-recognition and ATP-binding sites and is therefore solely responsible for activation of the recognised amino acid as its acyl adenylate through ATP hydrolysis (T. Stachelhaus et al., J. Biol. Chem. **270**, 1995, p.6163-6169).

**Table 1.**

| Highly conserved core motifs of catalytic domains of known peptide synthetases Source: M. Marahiel et al., Chem.Rev. **97**, 1997, p.2651-2673 | | |
|---|---|---|
| Domain | Core(s) Note: Former nomenclature is given in brackets | Consensus sequence |
| Adenylation | A1 | L(TS)YxEL |
| | A2 (core 1) | LKAGxAYL(VL)P(LI)D |
| | A3 (core 2) | LAYxxYTSG(ST)TGxPKG |
| | A4 | FDxS |
| | A5 | NxYGPTE |
| | A6 (core 3) | GELxIxGxG(VL)ARGYL |
| | A7 (core 4) | Y(RK)TGDL |
| | A8 (core 5) | GRxDxQVKIRGxRIELGEIE |
| | A9 | LpxYM(IV)P |
| | A10 | NGK(VL)DR |
| Thiolation | T (core 6) | DxFFxxLGG(HD)S(LI) |
| Condensation | C1 | SxAQxR(LM) (WY) xL |
| | C2 | RHExLRTxF |
| | C3 (His) | MHHxISDG(WV)S |
| | C4 | YxD(FY)AVW |
| | C5 | (IV)GxFVNT(QL) (CA)xR |
| | C6 | (HN)QD(YV) PFE |
| | C7 | RDxSRNPL |
| Thioesterase | TE | G(HY)SxG |

Very recently the first 3D structure of an adenylation domain of a peptide synthetase (PheA from GrsA) has been reported (E. Conti et al., EMBO J. **16**, 1997, p.4174-4183). This structure shows that almost all highly conserved core motifs are positioned around the active site where the substrates are bound. The main residues involved in building the substrate-binding pocket could also be assigned; they were found to be located between core motifs A3 and A6 and were not highly conserved.

The A-domain of a module is very important in determining the specificity of the module. "Specificity" of a module means that the module has a certain preference in recognising one amino acid above other amino acids or above another amino acid. Of course, also the concentration of each individual amino acid present near the module may play a role. If, for instance, the concentration of a specific amino acid is much higher than that of (most of) the other amino acids, the requirements for specificity may be somewhat less strict.

The so-called "thiolation domain" (T-domain, about 100 amino acids; also called peptidyl carrier protein (PCP)) is a domain located directly downstream of the adenylation domain. It forms an integral part of peptide synthetases, and is the site of 4'-PP cofactor binding and substrate acylation. Within the T-domain, 4'-PP is covalently bound to the sidechain of an invariant serine residue located within the highly conserved thiolation core motif (see table 1). If the T-domain in a module of the peptide synthetase does not carry its 4'-PP cofactor, no covalent binding of the aminoacyl substrate can take place and chain elongation will be impossible.

It has been found that in peptide synthetases known so far, every T-domain is converted from the inactive apo form to the active holo form by transfer of the 4'-PP moiety from Coenzyme A (CoA) to the sidechain of the above mentioned serine residue. This post-translational priming of each T-domain is mediated by peptide synthetase specific members of a recently discovered enzyme superfamily, the 4'-phosphopantetheinyl transferases. They utilise CoA as a common substrate, and appear to attain specificity through protein/protein interactions.

The Asp-Phe dipeptide synthetase as used in the method of the present invention comprises two minimal modules, respectively one minimal module at its N-terminal side recognising L-Asp and another minimal module at its C-terminal side recognising L-Phe. The term "minimal module" is used in the same meaning as given thereto by Stachelhaus et al., J. Biol. Chem. **270**, 1995, p.6163-6169.

Each of these minimal modules is composed of an adenylation domain (A-domain) and a thiolation domain (T-domain).

Moreover, the two minimal modules of the Asp-Phe dipeptide synthetases according to the invention are connected by a so-called condensation domain, which needs to be covalently bound to the polypeptide chain of the Phe-module, namely to the N-terminal part thereof. The condensation domain, however, does not need to be bound covalently to both minimal modules (i.e. to the modules recognising respectively L-Asp and L-Phe) because there is no requirement that these two minimal modules are located on a single polypeptide chain. The term "connected" therefore means that the condensation domain ensures that both minimal modules can operate concertedly.

In known peptide synthetases the condensation domains occur as a moderately conserved region. The condensation domain (conserved region) usually consists of about 400 amino acid residues and is known to be involved in the catalysis of non-ribosomal peptide formation. One of the conserved core motifs contains the catalytically active histidine residue. See T. Stachelhaus et al., J. Biol. Chem. **273**, 1998, p.22773-22781.

The Asp-Phe formed can be recovered from the reaction medium by any method available to the skilled man.

It is preferred, that the condensation domain in the dipeptide synthetase is connected to both minimal modules in such way that it is also covalently bound to the module recognising L-Asp. In such case the condensation domain is not only bound covalently to the N-terminal end of the L-Phe recognising module, but also to the C-terminal end of the L-Asp recognising module, and forms part of a single polypeptide chain comprising the L-Asp and L-Phe recognising modules.

A distinguishing feature of non-ribosomal peptide synthesis is the fact that the peptide formed on the template is covalently bound to the T-domain of the C-terminal module as a peptidyl-(4'-PP)-T-domain intermediate. Release of the peptide from this intermediate is assumed to take place either by intermolecular attack by water, resulting in net hydrolysis, or by intramolecular capture by a hydroxyl or amino group of the peptide chain itself, giving rise to a cyclic peptide product and of the peptide synthetase in the holo form. The first termination route yields a linear peptide with a free C-terminal carboxylic group (as should be the case for the Asp-Phe synthesis according to the present invention).

Because the Asp-Phe is present in an intermediate form bound to the template of the Asp-Phe dipeptide synthetase, it is advantageous to take additional measures for enhancing the release of the Asp-Phe from said template.

It is therefore particularly preferred that also a releasing factor is present for the Asp-Phe formed on the dipeptide synthetase. The term "releasing factor" as used here is intended to comprise any means, whether part of the synthetase or present in combination therewith, which enhance the releasing from the synthetase of the Asp-Phe formed on the synthetase.

All known bacterial and some fungal peptide synthetase modules that incorporate the last amino acid into the growing peptide chain show a region with a thioesterase-like function. These regions of approximately 250 amino acids are located at the C-terminal end of the amino acid recognising modules. These thioesterase-like regions are integrated regions which exhibit homology to thioesterase-like proteins, and therefore also are referred to as the thioesterase domain ((integrated) TE-domain). All these integrated TE-domains contain an active site serine residue, which is part of the core motif GxSxG (see table 1).

Recent work has given support for the theory that these integrated TE-domains are involved in the termination of the chain elongation and the product release. For instance, deletion of the complete TE-domain from the surfactin synthase led to a 97% reduction of the *in vivo* surfactin production (Schneider A., et al., Arch. Microbiol., **169**, 1998, p.404-410). Furthermore, it has been shown that replacing the integrated TE-domain from the C-terminus of module 7 of the surfactin synthase to the C-terminal ends of modules 4 and 5, resulted in the formation of the corresponding lipotetra- and pentapeptide. Also in this study the removal of the integrated TE-domain led to an almost complete reduction of peptide synthesis (Ferra F de, et al., J. Biol. Chem., **272**, 1997, p.25304-25309).

In a particularly preferred embodiment of the invention the releasing factor therefore is a protein which shows thioesterase-like functions and forms an integrated domain of the dipeptide synthetase at the C-terminus thereof.

In addition it is preferred that the Asp-Phe dipeptide synthetase, prior to the production of Asp-Phe, has undergone optimisation for its function by using one or more post-translational modifying activities. This is useful for achieving the most efficient non-ribosomal synthesis of Asp-Phe.

The term "post-translational modifying activities" for efficient non-ribosomal synthesis of Asp-Phe as used here is intended to comprise any activities which modify the dipeptide synthetase after its formation thereby positively affecting its Asp-Phe synthesising function.

In particular, in the production of Asp-Phe according to the present invention the post-translational modifying activity used is a 4'-phosphopantetheinyl (4'-PP) transferase. The 4'-PP transferase provides for effective conversion of the apo- to holo-enzyme of the peptide synthetase and by loading the 4'-PP cofactor to the serine side-chains in the core motif of the T-domains, and thereby increases the yield of Asp-Phe in the production thereof. Effective conversion of apo- to holo-enzyme is provided if in each of both T-domains of the Asp-Phe dipeptide synthetase at least 10% of the apo-enzyme is converted to the holo-form.

It is particularly preferred that in the production of Asp-Phe according to the invention also a non-integrated protein with thioesterase Type-II-like activity is present together with the dipeptide synthetase. As meant herein proteins having thioesterase Type-II-like activity are proteins with strong sequence similarities to type-II fatty acid thioesterases of vertebrate origin. Such non-integrated protein with thioesterase Type-II-like activity is different from the integrated thioesterase (TE-domain). Recent work (Schneider et al., Arch. Microbiol. (1998), 169, 404-410) has shown that deletion of a gene encoding such non-integrated protein with thioesterase Type-II-like activity from the surfactin synthase operon leads to an 84% reduction of peptide production. It is suggested that the non-integrated protein with thioesterase Type-II-like activity enhances production of non-ribosomal peptides, possibly by reactivation through liberation of mischarged modules that are blocked with an incorrect aminoacyl group or an undesired acyl group at the 4'-PP cofactor.

The genes coding for the non-integrated proteins with thioesterase Type-II-like activity can be positioned at the 5'- or 3'-end of the peptide synthetase encoding operon. These proteins have molecular masses of 25-29 kDa, are about 220-340 amino acid residues in length, and carry the sequence GxSxG which is presumed to form the active site. It is noticed that in almost all of the prokaryotic peptide synthetase coding operons known so far, such distinct genes have been detected.

In the production of Asp-Phe according to the present invention the Asp-Phe dipeptide synthetase is preferably present in living cell-material of a micro-organism, and glucose, L-Asp and/or L-Phe are being fed to said fermentor, and the Asp-Phe formed is then recovered. As used in this context the term "glucose" is intended to cover glucose and any other energy source necessary for the regeneration of ATP in the living cell material and for the maintainance energy required for said living cell material. The glucose (or other energy source), moreover, is used as starting material for the production of any L-Asp and/or L-Phe to be produced in the living cell in the course of the process of the invention.

The skilled man, of course, will be aware that the feeding of glucose, L-Asp and/or L-Phe is to be done under appropriate conditions of temperature and pH, including as required the presence of an appropriate nitrogen source, salts, trace elements, and other organic growth factors as vitamins and amino acids, etc. to the fermentor or other type of (enzyme) reactor which is used for the production of Asp-Phe. The Asp-Phe formed is recovered. Such recovery may take place during the process or at the end thereof.

The living cell-material may be present in any appropriate form as available to the skilled man. For instance, whole cells may be used as such or in immobilised form. The micro-organism may be any kind of micro-organism wherein the Asp-Phe dipeptide synthetases according to the invention can stably be expressed. Suitable micro-organisms are, for instance, micro-organisms which
(a) are producing peptides via non-ribosomal synthesis, for instance, bacteria as *Streptomyces* species, *Bacillus* species, *Actinomyces* species, *Micrococcus* species, *Nocardia* species, or fungal species as *Tolypocladium* species, *Fusarium* species, *Penicillium* species, *Aspergillus* species, and *Cochliobolus* species; or
(b) are capable of producing amino acids, in particular L-Asp and/or L-Phe, preferably on industrial scale, for instance, *Escherichia* species, e.g. *E.coli,* and *Corynebacterium* species, e.g. *C.glutamicum.*

The micro-organisms may be grown, under conditions which can easily be found by the skilled man, in a fermentor, and production of the Asp-Phe then can be carried out in the same or in another fermentor. As meant herein the fermentor may be any type of fermentor or other types of (enzyme) reactor known to the skilled man.

In the method for the production of Asp-Phe according to the present invention it is preferred that the micro-organism is first grown in a fermentor to reach a predetermined cell density before the expression of the Asp-Phe dipeptide synthetase is switched on and feeding of the glucose, L-Asp and/or L-Phe for the synthesis of the Asp-Phe dipeptide is started.

The skilled man can easily determine the growth of the micro-organism, e.g. by measuring its optical density (O.D.), and find the most appropriate level of cell density. To prevent any negative effect on the growth of the micro-organism, growth phase and Asp-Phe synthetase productio phase are preferably uncoupled. Such uncoupling can be achieved by expressing the gene for the Asp-Phe synthetase from an inducible, tightly regulable, promoter. The expression of the Asp-Phe dipeptide synthetase is preferably switched on by addition of a specific chemical component (inducer) or by changing the physical conditions, e.g. the temperature, pH or dissolved oxygen pressure, after a predetermined level of cell density has been reached. The expression is assumed to be switched-on as compared to the non-induced state, if the expression level of the Asp-Phe dipeptide synthetase is raised at least by a factor of 10.

Then also the feeding of substrates, etc. in amounts as required, is started, and production of Asp-Phe starts.

Most preferably the micro-organism is an L-phenylalanine producing micro-organism and, apart from required amounts of salts and trace elements etc., only glucose and L-Asp are being fed. L-Phe producing micro-organisms are well-known. For instance, *E. coli* and *Corynebacterium* species are being used for L-Phe production. By expressing an Asp-Phe dipeptide synthetase in such micro-organisms availability of L-Phe in the micro-organism is provided for, and only glucose, an appropriate nitrogen source, organic growth factors, salts and trace elements, etc., as well as L-Asp, should be supplied as required.

In particular the micro-organism used is an *Escherichia* or a *Bacillus* species.

Best results are obtained if the micro-organism used is a strain with reduced protease activity for Asp-Phe or lacking such activity towards Asp-Phe. By using such strains degradation of Asp-Phe formed is prevented. Any suitable strain which lacks protease activity (either naturally or because the activity of the protease has been lowered substantially or has been removed completely) may be used.

Moreover, if the synthesis of Asp-Phe occurs in a micro-organism, also additional measures can be taken for improving the permeation of the Asp-Phe formed into the reaction medium outside the micro-organism and recovering the Asp-Phe therefrom after separation of the micro-organism from the reaction medium. Similarly, also additional measures may be taken for improving the uptake of glucose and/or L-Asp and/or L-Phe.

In an even more preferred embodiment of the invention the micro-organism used also contains a suitable export system for Asp-Phe formed and/or one or more suitable uptake system(s) for glucose and/or L-Asp and/or L-Phe. Using a suitable export system will ensure achieving more efficient secretion of the Asp-Phe formed. The secretion meant here is the secretion of Asp-Phe formed in the micro-organism into the extracellular environment. Efficient secretion of Asp-Phe is important for improving the recovery yield of Asp-Phe and for maintaining the activity of the Asp-Phe dipeptide synthetase at a suitable level as well as for preventing intracellular degradation of Asp-Phe. Moreover, the down-stream processing for Asp-Phe secreted is more easy.

Similarly, the presence of suitable uptake system(s) will improve the coupling efficiency to Asp-Phe.

In the foregoing paragraphs *in vivo* non-ribosomal synthesis methods for Asp-Phe have been described. They all are characterised in that living cell material is used and ATP regeneration takes place in this cell material. The present invention can also be carried out *in vitro.* As used herein, *in vitro* systems are characterised in that the Asp-Phe dipeptide synthetase is not present in living cell material; it may, however, be present in any other environment, for instance in permeabilised cells, cell-free extract, or as an isolated dipeptide synthetase. In such case regeneration of ATP does not take place in living cell material used for the synthesis of Asp-Phe, and special measures for supply of ATP in an effective amount have to be taken.

In a preferred embodiment of the invention, the production of Asp-Phe is carried out *in vitro* in an enzyme reactor, while ATP is supplied, and L-Asp and/or L-Phe are being fed, and the Asp-Phe formed is recovered.

In order to improve the economic feasibility of the process it is particularly preferred to increase the yield of Asp-Phe per mole of ATP supplied for the synthesis of Asp-Phe. This can be achieved by in situ ATP regeneration from the AMP formed out of the ATP in the consecutive adenylation and thiolation reactions.

Therefore, in a preferred mode of the invention, the supply of ATP is provided in part by an in situ ATP-regenerating system.

Various ATP regenerating systems (which in the literature are also being referred to as ATP generating systems) are known to the skilled man. As ATP regenerating systems both whole cell systems (e.g. yeast glycolysis systems) or isolated ATP regenerating enzymes, for instance adenylate kinase combined with acetate kinase, may be used. A very elegant ATP regeneration system has been described by T. Fujio et al. (Biosci., Biotechnol., Biochem. **61**, 1997, p.840-845). They have shown the use of permeabilised *Corynebacterium ammoniagenes* cells for regeneration of ATP from the corresponding monophosphate (AMP) coupled to an ATP-requiring reaction in permeabilised *E. coli* cells. In this elegant way (cheap) glucose can be supplied as an energy source instead of most of the ATP.

Therefore, the ATP-regenerating system is preferably present in a permeabilised micro-organism. This permeabilised micro-organism present in the (enzyme) reactor used ensures that an effective amount of adenosine-triphosphate (ATP) will always be present and available during the Asp-Phe production according to the present invention.

### DNA FRAGMENTS ENCODING AN Asp-Phe DIPEPTIDE SYNTHETASE, ETC.

The present invention also relates to novel DNA fragments encoding an Asp-Phe dipeptide synthetase.

These novel DNA fragments or combination of DNA fragments code for a non-ribosomal Asp-Phe dipeptide synthetase, which synthetase comprises two minimal modules connected by one condensation domain wherein the N-terminal module of these modules is recognising L-aspartic acid and the C-terminal module of these modules is recognising L-phenylalanine and is covalently bound at its N-terminal end to the condensation domain, and wherein each of these minimal modules is composed of an adenylation domain and a 4'-phosphopantetheinyl cofactor containing thiolation domain.

The term "DNA-fragment or combination of DNA fragments" as used herein is understood to have its broadest possible meaning. The term first of all relates to the composite biological material (on one or more DNA-fragments) as mentioned herein-above and coding for the minimal modules for Asp and Phe in the correct order and for the condensation domain, each coding sequence being surrounded by any transcription and translation control sequences (e.g. promoters, transcription terminators) and the like which may be suitable for the expression of the Asp-Phe dipeptide synthesising activity. The control sequences may be homologous or heterologous, and the promoter(s) present in the DNA may be constitutive or inducible.

The term "DNA-fragment" as used herein is further understood to code, in addition to coding for the Asp and Phe minimal modules and the condensation domain, for the activities of the other domains, e.g. TE-domains. Furthermore, these fragments may code for activities which are not located on the Asp-Phe dipeptide synthetase polypeptide itself, such as non-integrated thioesterase Type-II-like proteins, and other activities co-operating concertedly with the Asp and Phe minimal modules.

The term "DNA-fragment" as used herein is also understood to comprise gene structures comprising DNA fragments as described herein-above. More precisely, a gene structure is to be understood as being a gene and any other nucleotide sequence which carries the DNA-fragments according to the invention. Appropriate nucleotide sequences can, for example, be plasmids, vectors, chromosomes, or phages. The gene structures may exist either as (part of) an autonomously replicating vector in single or multicopy situation, or integrated into the chromosome in single or multicopy situation.

The gene structure is also to be understood as being a combination of the above-mentioned gene carriers, such as vectors, chromosomes and phages, on which the DNA-fragments according to the invention are distributed. For example, the Asp-Phe dipeptide synthetase encoding DNA-fragment can be introduced into the cell on a vector and the non-integrated thioesterase Type-II-like protein encoding DNA-fragment can be inserted into the chromosome. In addition, a further DNA-fragment can, for example, be introduced into the cell using a phage. These examples are not intended to exclude other combinations of DNA-fragment distributions from the invention. The DNA-fragments according to the invention may be introduced into the micro-organism at a sufficiently high copy number, for instance of up to 50 copies.

A detailed discussion of the Asp-Phe dipeptide synthetase and the two minimal modules comprised therein already has been given in the preceding parts of this patent application.

The construction of the DNA fragments according to the present invention is not self-evident, although terms like "module", "domains", etc. misleadingly might suggest that much is known about the functional boundaries thereof. Such detailed information which would enable rational design of (mutant, non-natural) peptide synthetases, however, is not yet available. Nevertheless, recently various techniques for construction of mutant peptide synthetases have been described in literature. Methods for construction of mutant peptide synthetases described in literature are:

De Ferra et al. (J. Biol. Chem., **272**, 1997, p.25304-25309) have described a method for producing truncated peptides of a predicted sequence by replacement of the integral TE-domain of the surfactin synthetase from the C-terminal module to the C-terminal end of different internal modules. This technique alone, however, is not suitable for the construction of an Asp-Phe dipeptide synthetase module because the natural order of two consecutive Asp and Phe modules is not known to exist in nature (neither at the N- or C-terminal end of any natural synthetase, nor as an internal sequence of any naturally occurring synthetase).

Another *in vivo* technique described for engineering peptide synthetases is the so-called programmed alteration within the primary structure of a peptide product. Basis for this method is the replacement of one module by another on the genetic level. This technique has been described in general by A. Schneider et al., Mol. Gen. Genet., **257**, 1998, p.308-318). In this way amino acid-activating minimal modules could be exchanged successfully *in vivo* between multi-modular peptide synthetases of heterologous origin, and micro-organisms could be obtained which indeed produce non-ribosomal peptides with a different primary structure from the peptides produced without such alteration.

If this technique would be applied for the construction of an Asp-Phe dipeptide synthetase, in principle two options would be available, each starting from a dipeptide synthetase, namely having a sequence of two modules comprising either (i) Asp and XXX, the latter representing any other amino acid than Phe, or (ii) YYY and Phe, the former representing any other amino acid than Asp. In those dipeptide synthetases the DNA coding for the XXX module should be replaced by DNA coding for the Phe module, or the DNA coding for the YYY module by DNA coding for the Asp module.

Other methods for construction of peptide synthetases have been described in EP-A-0637630. In said patent application a method is suggested whereby, next to alteration of the substrate specificity by substitution of (part of) modules, also modules can be deleted or inserted into the synthetase chain. "Specificity" of a module means that the module has a certain preference in recognising one amino acid above other amino acids or above another amino acid.

It is a distinctive feature of the above peptidase engineering methods that homologous recombination events are used to bring about the desired changes in the genomic DNA in the native peptide producing micro-organism. Because the homologous recombination events take place in the native peptide producing micro-organism, these methods would have the advantage that all the native host enzymes and relevant regulatory elements are present in principle.

However, homologous recombination through use of the native non-ribosomal peptide producer suffers from several severe drawbacks. The most serious of these drawbacks is that it is often tedious and technically difficult, especially when applied to slow-growing micro-organisms with poorly developed transformation systems or which are lacking in other genetic tools. Other drawbacks are that the native non-ribosomal peptide producing micro-organisms often do not have a history of safe use on industrial scale, are no production organisms for L-Phe and/or L-Asp, and have unknown fermentation characteristics. Moreover, all these methods result in cells having only a single copy of the DNA-fragment coding for the desired peptide synthetase. Therefore, none of these *in vivo* engineering methods are suitable for the preparation of the novel Asp-Phe dipeptide synthetase according to the invention and use thereof for the industrial production of Asp-Phe.

The present inventors now have found that the Asp-Phe dipeptide synthetase can be readily obtained by use of *in vitro* engineering techniques. So far no *in vitro* engineering techniques for the construction of peptide synthetases have been described. Detailed protocols for the construction of Asp-Phe dipeptide synthetases according to the invention can be found in the experimental part of this application.

The Asp-Phe dipeptide synthetase encoding DNA-fragment can be constructed *in vitro* from an Asp-XXX or YYY-Phe (with XXX and YYY having the meaning as described above) dipeptide synthetase encoding DNA-fragment (or partial sequences for such synthetase encoding fragments as occurring in a naturally existing peptide synthetase). This was accomplished starting from an Asp-Leu (Leu = leucine) dimodular peptide synthetase encoding DNA-fragment which was obtained from the *Bacillus subtilis* ATCC 21332 surfactin synthetase A gene (srfA-B) by PCR method. The Leu minimal module encoding DNA-fragment thereof then was replaced by a DNA-fragment (obtained by PCR method) from the *Bacillus brevis* ATCC 8185 tyrocidine A synthetase gene coding for a Phe minimal module (*tyc*A). Then an integral TE-domain was added to the C-terminal end of the Asp-Phe encoding DNA-fragment by replacement of the thiolation domain of the Phe module by a PCR-fragment coding for the *srf*A-C thiolation and TE domain. This construction was done in such a way that the DNA encoding the additional TE domain was fused in-frame with the gene encoding the Asp-Phe synthetase. As a result the TE-domain forms an integrated part of the Asp-Phe synthetase produced. In the experimental part of this application this TE-domain containing Asp-Phe synthetase will be referred to as Asp-Phe-TE.

After the construction, the encoding DNA-fragments were introduced into a suitable host micro-organism. Suitable host micro-organisms are, for instance, *E. coli* and *Bacillus* species. After cultivation of these micro-organisms under inducing conditions, cells were lysed and the synthetases produced were purified by IMAC (Immobilised Metal Affinity Chromatography). The purified enzyme preparations were used for different experiments to prove the formation of Asp-Phe.

### Preferred DNA fragments

The following preferred aspects of the DNA fragments encoding the Asp-Phe dipeptide synthetase according to the invention closely correspond to the aspects discussed in the previous parts of this specification regarding the preferred methods for the production of Asp-Phe.

For the DNA fragments encoding the Asp-Phe dipeptide synthetase according to the invention it is especially preferred that the condensation domain in the encoded dipeptide synthetase is connected to both minimal modules in such way that it is also covalently bound to the module recognising L-aspartic acid.

In particular it is preferred that the DNA fragment or combination of DNA fragments encoding the dipeptide synthetase also code for a releasing factor for the Asp-Phe formed on that dipeptide synthetase. The term "releasing factor" is used in the same meaning as it has been used in the previous part of the specification.

In a more particularly preferred embodiment of the present invention, the DNA fragment or combination of DNA fragments encoding the Asp-Phe dipeptide synthetase is/are also coding for a protein which shows thioesterase-like functions and forms an integrated domain of the dipeptide synthetase at the C-terminus thereof. For an explanation of the terms "integrated domain" etc., reference is made to earlier parts of the present application.

In addition, the synthetase encoding DNA fragment or combination of DNA fragments preferably also express(es) one or more post-translational modifying activities for efficient non-ribosomal synthesis of Asp-Phe on the synthetase. The terms "post-translational modifying activities", etc. are used in the same meaning as they have been used in the previous part of the specification.

In particular, the post-translational modifying activity expressed by the DNA fragment or combination of DNA fragments is a 4'-phosphopantetheinyl (4'-PP) transferase activity. The formation of this activity provides for effective conversion of apo- to holo-enzyme. Effective conversion of apo- to holo-enzyme, etc. already has been explained in the previous part of the specification.

It is particularly preferred that the DNA fragment or combination of DNA fragments also code(s) for a non-integrated protein with thioesterase Type-II-like activity. The term "non-integrated protein with thioesterase Type-II-like activity" is used in the same meaning as it has been used in the previous part of the specification.

### Micro-organisms

The invention further relates to micro-organisms containing a DNA fragment or combination of DNA fragments according to the invention, and in particular to such micro-organisms which are capable of producing L-Asp and/or L-Phe. In particular, the micro-organism is an *Escherichia coli* or *Bacillus* species.

### Asp-Phe dipeptide synthetases

The present invention finally also relates to novel Asp-Phe dipeptide synthetases. The terms and expressions used hereinafter with respect to the Asp-Phe dipeptide synthetase all have the same meaning as explained herein-above.

The non-ribosomal Asp-Phe dipeptide synthetases according to the present invention are characterised in that they comprise two minimal modules connected by one condensation domain wherein the N-terminal module of these modules is recognising L-aspartic acid and the C-terminal module of these modules is recognising L-phenylalanine and is covalently bound at its N-terminal end to the condensation domain, and wherein each of these minimal modules is composed of an adenylation domain and a 4'-phosphopantetheinyl cofactor containing thiolation domain.

In particular, the condensation domain in the dipeptide synthetases is connected to both minimal modules in such way that it is also covalently bound to the module recognising L-aspartic acid.

Preferably, the Asp-Phe dipeptide synthetase also comprises a releasing factor for the Asp-Phe formed on that dipeptide synthetase.

Most preferably, the releasing factor is a protein which shows thioesterase-like functions and forms an integrated domain of the dipeptide synthetase at its C-terminus.

The invention hereinafter now will be clarified further in the experimental part, but will in no way be restricted to the experiments shown. Amino acids used all were enantiomerically pure L-amino acids.

### EXPERIMENTAL PART

### General procedures

Standard molecular cloning techniques such as DNA isolation, gel electrophoresis, enzymatic restriction modifications of nucleic acids, *E.coli* transformation etc., were performed as described by Sambrook et al., 1989, "Molecular Cloning: a laboratory manual", Cold Spring Harbor Laboratories, Cold Spring Harbor, New York and Innis et al., 1990, "PCR protocols, a guide to methods and applications" Academic Press, San Diego. Synthetic oligo deoxynucleotides were obtained from MWG-Biotech AG, D-Ebersberg. DNA sequence analyses were performed on an Applied Biosystems ABI 310 genetic analyzer, according to supplier's instructions. Sequencing reactions were carried out by the chain termination method with dyelabelled dideoxy terminators from the PRISM ready Reaction DyeDeoxy Terminator cycle sequencing kit with AmpliTaq FS polymerase (Applied Biosystems).

### Construction of plasmid pasp-leu-His₆

A 4934 bp fragment comprising regions from the *srf*B locus from chromosomal *Bacillus subtilis* ATCC 21332 DNA was amplified (PCR) using the following primers:

5′ TAA GCA TGC TGC TTT CAT CTG CAG AAA C (5′ asp-leu-
SphI- srfB2), and
3′ AAT GGA TCC TTC GGC ACG CTC TAC (3′ asp-leu-BamHI-
srfB3).

Correct size of the amplified fragment was confirmed by agarose gel electrophoresis.

The fragment (20 µg) was digested with 1 unit of the enzymes *Bam*HI/*Sph*I (37°C, 16 h) to generate terminal restriction sites.

Plasmid pQE70 (provided by Qiagen, D-Hilden) (10 µg) was digested with the same enzymes and subsequently incubated for 1 hour with 1 unit Alkaline Phosphatase (37°C). Complete digestion was confirmed by transforming 1 µL of the linearised plasmid DNA into competent cells of *E.coli* XL1 blue. The two fragments were subsequently ligated in a ligation reaction (10 µL) in a vector/insert ratio of 1:3 with 1 unit of T4-DNA-ligase enzyme (16°C, 16 h).
1 µL of the ligation mixture was used to transform 40 µL competent cells of *E.coli* XL1 blue (Stratagene, D-Heidelberg) by electroporation. The transformants were selected on 2x YT agar plates containing Ampicillin (100 µg/mL). Analysis of 48 transformants resistant to ampicillin revealed that 4 of them had inserted a ca. 5000 bp fragment. Correct insertion was confirmed using restriction enzyme digestion analysis and terminal sequencing of the insert. A correct clone designated *pasp-leu-His*_{**6**} was used for further investigations.

### Construction of plasmid pasp-phe-His₆

Plasmid *pasp-phe-His*_{*6*} was constructed from plasmid *pasp-leu-His*_{*6*} as follows.

A 1894 bp chromosomal DNA-fragment from *Bacillus brevis* ATCC 8185 DNA was amplified (PCR) using the following primers:

5′ ATT TGG TCA CCA ATC TCA TCG ACA A (5′ BstEII-TycA-
NLID), and
5′ ATA GGA TCC TGT ATT CGT AAA GTT TTT C (3′-PheAT-
BamHI).

Correct size of the fragment was confirmed using agarose gel electrophoresis.

The fragment was digested with 1 unit of enzyme *Bam*HI and incubated at 30°C for 4 hours.
Subsequently 1 unit of enzyme *BstE*II was added and incubated for another 4 hours at 60°C.

Plasmid *pasp-leu-His*₆ was digested in the same way and subsequently incubated for 1 hour with 1 unit of Alkaline phosphatase. The vector portion (ca. 6,5 kb) was separated from other DNA fragments by agarose gel electrophoresis and repurified. Complete digestion was confirmed as before with linearised *pasp-leu-His*₆. The two fragments were ligated in a equimolar ratio for 5 hours at 16°C using 1 unit of T4-ligase enzyme. 1 µL of the ligation mixture was used for electroporation of *E.coli* XL1 blue competent cells. Transformants were selected on 2x YT agar containing Ampicillin (100 µg/mL). Analysis of transformants revealed that 1 out of 90 clones had inserted a fragment of ca. 2000 bp. Correct insertion was confirmed using restriction enzyme digestion analysis and terminal sequencing of the insert.

The correct clone was designated *pasp-phe-His*₆. In contrast to the peptide synthetase encoding gene on plasmid *pasp-leu-His*₆, the peptide synthetase encoding gene on plasmid *pasp-phe-His*₆ is a hybrid gene obtained by exchanging the DNA-fragment coding for the second (Leu) minimal module (A- and T-domain), for a DNA-fragment coding for a Phe minimal module.

### Construction of plasmid pasp-phe-TE-His₆

Plasmid *pasp-phe-*TE*-His*₆ was constructed from plasmid *pasp-phe-His*₆.

A 910 bp chromosomal DNA-fragment from *Bacillus subtilis* ATCC 21332 DNA was amplified (PCR) using the following primers:

5′ ATA ATC GAT AAT CGC ACA AAT ATG GTC (5′ TE-srfC1-
ClaI) and
3′ ATA  AGA TCT AAC AAC CGT TAC GGT TTG TGT (3′ int TE-
srfC1-BglII).

Correct size of the fragment was confirmed using agarose gel electrophoresis.

The fragment was digested with 1 unit of enzyme *Cla*I for 4 hours at 37°C, before adjusting buffer conditions and digesting with 1 unit of enzyme *Bg*lII (4 hours, 37°C).
Plasmid *pasp-phe-His*₆ was digested with enzyme *Cla*I (4 h, 37°C) and subsequently with *Bam*HI (4 h, 37°C) before the linearised plasmid was incubated for one hour with 1 unit of Alkaline phosphatase. The vector portion (ca. 8 kb) was separated from other DNA-fragments by agarose gel electrophoresis and repurified.

Control of complete digestion, ligation, electroporation and selection of transformants was established as described before.

Two of the analysed transformants were shown to contain the desired DNA-fragment. Correct insertion of the 900 bp fragment was confirmed by restriction enzyme analysis and terminal sequencing of the insert.

A correct clone was designated *pasp-phe-*TE*-His*₆. In contrast to the peptide synthetase encoding gene on plasmid *pasp-phe-His*_{*6*}*,* the peptide synthetase encoding gene on plasmid *pasp-phe-TE-His*₆ contains a second fusion site located between the DNA coding for the adenylation domain and thiolation domain of the second (Phe) minimal module. The C-terminal T-TE domains resemble the native C-terminus of the Surfactin synthetase *srf*C.

### Expression of the peptide synthetases asp-leu-His_{₆,} asp-phe-His_{₆} andasp-phe-TE-His₆

1 µL of each constructed plasmid were transformed in *E.coli* BL21/p*gsp* competent cells. Strain BL21 lDE3 was obtained from Stratagene, D-Heidelberg. Plasmid p*gsp,* which is based on plasmid pREP4 (obtained from Qiagen, D-Hilden), contains the *gsp* gene (the 4'-PP transferase gene from the Gramicidin S-biosynthesis operon from *Bacillus brevis* ATCC 9999) under control of the T7 promoter.

Transformants were selected on 2x YT agar plates containing Ampicillin (100 µg/mL ) and Kanamycin (25 µg/mL). Several colonies were used to inoculate 4 mL of 2x YT liquid medium (containing in addition 10 mM MgCl₂) and incubated at 37°C for 16 hours. These 4 mL cultures were subsequently used to inoculate 400 mL of the same medium. Cells were grown at 30°C in a waterbath shaker (250 rpm). After 3-4 hours the cells reached an optical density of 0,7 (OD₆₀₀ₙₘ) and were induced by the addition of 200 µM IPTG. Cells were incubated for an additional 1,5 hours before being harvested.

Expression of recombinant proteins was confirmed by SDS-PAGE comparing protein samples taken at the time of induction and 1,5 hours later.

In crude cell extracts from BL21*/pgsp/pasp-leu-His*₆ and BL21*/*p*gsp/pasp-phe-His*₆ expression of an inducible protein of ca. 180 kDa was confirmed. From crude cell extracts of BL21/p*gsp/pasp-phe-TE-His*₆ expression of an inducible protein of ca. 200 kDa could be shown.

From cultures expressing the correct recombinant proteins glycerol stocks were prepared and stored at -80°C.

### Purification of the recombinant proteins Asp-Leu-His₆, Asp-Phe-His₆ and Asp-Phe-TE-His₆

800 mL cultures of BL21 */pgsp/pasp-leu-His*₆, BL21*/pgsp/pasp-phe-His*₆ and BL21*/pgsp/pasp-phe-TE-His*₆ treated as described in "Expression of the peptide synthetases were centrifuged at 5000 rpm for 5 minutes and resuspended in 30 mL/L culture of buffer A (50 mM HEPES, 300 mM NaCl, pH 8,0). Cell suspensions were used directly or were stored at -20 °C till usage. Cell lysis was established using two French press passages at a working pressure of 12000 psi.

Directly after cell lysis PMSF was added to a final concentration of 1 mM. After centrifugation of the cell lysates at 10000 rpm for 30 minutes, the supernatant was combined with 1% (v/v) buffer B (50 mM HEPES, 300 mM NaCl, 250 mM Imidazol, pH 8,0). Protein solutions were applied on a Ni²⁺-NTA-agarose column (Qiagen, D-Hilden) previously equilibrated with 1% (v/v) buffer B. Flow rate was 0,75 mL/min. After the non-His₆-tagged proteins had passed through the column, it was washed with 1% buffer B for another 10 min before a linear gradient was applied (30 min to 30% B, an additional 10 min to 100% B). All three proteins eluted at a concentration of about 5% buffer B (15 mM Imidazol) and were collected as 2 mL fractions.

Fractions containing the recombinant proteins were detected using the Bradford reagent, by the absorption at 595 nm. These fractions were pooled and dialysed against a buffer containing 50 mM HEPES, 100 mM NaCl, 10 mM MgCl₂ and 5 mM DTE for 16 hours. After dialysation protein concentrations were again determined.

Till further usage proteins were stored at-20°C after addition of glycerol to 10% (v/v).

From 1 L culture approximately 5 mg of each pure recombinant protein could be obtained. Grade of purification was estimated to be 95% by SDS-PAGE.

### Analysis of enzymatic activity

### ATP - PPᵢ - exchange reaction:

Specificity of amino acid activation was determined indirectly by incorporation of labelled ³²PPᵢ into ATP during reverse reaction (Lee, S.G. & Lipmann, F.; Tyrocidine synthetase system; *Methods Enzymol. **43**,* 1975, p.585-602). For this purpose 20 pmol of each enzyme was incubated with 1 mM amino acid, 1 mM ATP, 0,1 mM PPᵢ, 50 mM HEPES, 100 mM NaCl and 10 mM MgCl₂ and 2 mCi ³²PPᵢ at 37°C in a total volume of 100 µL. Reactions were quenched after 10 min by adding 500 µL of a solution containing 100 mM NaPPᵢ, 560 mM perchloric acid and 1,2% (w/v) active charcoal. The mixture was centrifuged at 13000 rpm for 1 min. The pellet was washed and resuspended twice with 1 mL H₂O.

Incorporation of labelled ATP (adsorbed to the charcoal) was detected by measuring radioactivity of the precipitate.
Asp-Leu-*His*₆ was shown to activate Asp and Leu exclusively. *K*_{*M*} values for Asp and ATP were determined to be 3,5 mM and 0,9 mM respectively. *K*_{*M*} values for Leu and ATP were detected to be 0,3 mM and 0,6 mM, respectively.

Asp-Phe-*His*₆ and Asp-Phe-TE-*His*₆ were shown to activate both Phe and Asp. The *K*_{M} value for Phe was determined to be about 50 µM.

The amino acid activation patterns of Asp-Phe-*His*₆ and Asp-Phe-TE-*His*₆ were found to be identical.

### Covalent binding of amino acids to the Asp-Phe dipeptide synthetase:

Quantity of holo-enzyme formation was determined by measuring the amount of labelled Asp, Leu and Phe, that could be covalently bound to one equivalent of the purified proteins Asp-Leu-*His*₆, Asp-Phe-*His*₆ and Asp-Phe-TE-*His*₆. (Lee, S.G.; see above).

50 pmol of each enzyme was incubated with 2 mM ATP, 50 mM HEPES, 100 mM NaCl, 10 mM MgCl₂ and 100 pmol of ¹⁴C labelled amino acid (Asp and Leu or Asp and Phe respectively) at 37°C. After 30 min the reaction was quenched by the addition of 1 mL of 10% TCA and 5 mg/mL BSA and subsequently stored at 0°C for another 30 min. The protein precipitates were collected by centrifugation (30 min at 13000 rpm) and washed twice with 10% TCA. The washed precipitates were dissolved in 50% performic acid and used to measure incorporation of labelled amino acid.

Asp-Leu-*His*₆ could be labelled with Asp and Leu to a degree of approximately 20-25%. Asp-Phe-*His*₆ and Asp-Phe-TE-*His*₆ could be labelled with Asp to a degree of only 10-15%. The incorporation of Phe reached a level of approximately 50%.

### Indirect proof of formation of the dipeptides Asp-Leu and Asp-Phe:

Covalent binding of the constituent amino acids to the dipeptide synthetases was shown by kinetic experiments using radioactively labelled Asp. In a first assay 0,85 µM Asp-Leu-*His*₆ were incubated with 2 mM ATP, 2,8 µM Asp (¹⁴C, 56 nCi) in a buffer (50 mM HEPES, 10 mM MgCl₂, 100 mM NaCl, pH 8,0) at 37 °C. At regular time intervals samples were taken and treated as indicated in the previous paragraphs; radioactivity (of Asp covalently bound to the enzyme) was measured in each sample. After 4 minutes. the amount of incorporated labelled Asp started levelling-off to reach a maximum a few minutes later if no second amino acid was added. If, however, 1,5 µM of Leu were added after 4 minutes, a further strong, but temporary, increase of radioactivity was observed, which sharply decreased after about 5 minutes to a level below the maximum observed when no second amino acid was added.

In another experiment 0,5 µM Asp-Phe-*His*₆ were incubated in the same way as Asp-Leu-*His*₆ before. In this case the addition of 0,1 mM Phe after about 5 minutes resulted in a similar temporary increase of covalently bound radioactive Asp, as described above. However, the addition of Leu instead of Phe did not lead to such temporary increase.

This clearly shows that peptide bond formation takes place on each of these peptide synthetases. Furthermore, the results show that the peptides formed on the peptide synthetases are being released therefrom.

### Direct proof of formation of the dipeptide Asp-Phe:

Formation of the dipeptide Asp-Phe was proven by a series of experiments, using different analytical techniques, namely thin layer chromatography (TLC) with radio-active detection, high performance liquid chromatography and mass spectroscopy.

In a first set of experiments (method to determine Asp-Phe released from the dipeptide synthetase) 100 pmol of Asp-Phe-*His*₆ were incubated in a total volume of 200 µl with lmM of ATP, 1 mM Phe and 1.25 µM ¹⁴C-labeled Asp in a buffer (50 mM HEPES, 20 mM MgCl₂, pH 8) for 6 hours at 37 °C. Control experiments carried out were done omitting one of ATP, Phe or enzyme.

In a further, analogous, set of experiments the same amount of said enzyme was incubated under identical conditions with 1 mM Asp and 1.25 µM ¹⁴C-labeled Phe (instead of Phe and ¹⁴C-labeled Asp). Also the appropriate blanks were carried out.

To finish the reactions, 100 µl of n-butanol were added to precipitate the enzyme which then was removed. The remaining clear solutions were evaporated and replenished to a volume of 20 µl with 10 vol% of methanol in water. An appropriate volume of each of these samples was applied to a silica TLC plate, which then was developed using a solution of butanol/acetic acid/ethyl acetate/water 1:1:1:1 (v/v/v/v) as the eluent. After elution the plate was dried and an X-ray film was put on top of it for 2 to 5 days of exposure. Finally the film was processed to show spots of radioactively labeled compounds.

Only if Asp, Phe, ATP and enzyme were present in the reaction mixture a spot could be detected that had the same retention factor (R_{f}) as Asp-Phe.

In a second set of experiments (method to determine dipeptide synthetase bound Asp-Phe) 1 nmol of Asp-Phe-*His*₆ were incubated in a total volume of 600 µl with lmM of ATP, 1 mM Asp and 1 µM ¹⁴C-labeled Phe in a buffer (50 mM HEPES, 20 mM MgCl₂, pH 8) at 37 °C.

Reactions were stopped after 10, 20 and 30 minutes, respectively, by the addition of 300 µl of 20% (weight) aqueous trichloroacetic acid (TCA) and the mixtures were cooled to 4 °C. All further steps were carried out at 4 °C.

The precipitated protein was collected by centrifugation, washed once with 500 µl of 10% aqueous TCA, then with 1 ml of a 3:1 (v/v) mixture of diethyl ether and ethanol, and finally with 1 ml of diethyl ether. Next the pellet was dried for 15 minutes at 37 °C, before being resuspended in 200 µl of 100 mM of an aqueous solution of potassium hydroxide under vigorous shaking. The mixture then was treated for 0.5 hours at 70 °C to release any thioester bound dipeptide from the enzyme. To separate the released dipeptide form the protein, the solution was replenished to 1 ml with methanol. This solution was centrifuged for 30 minutes at 4 °C and the supernatant was evaporated *in vacuo* for 3 hours at room temperature. The obtained pellet was finally resuspended in 25 µl of 10% aqueous methanol.

Analysis then was done by TLC with radio-active detection as described in the previous set of experiments. A clear spot of the dipeptide Asp-Phe could be observed in each of the three samples. The intensity of these spots increased significantly for the samples taken at 10, 20 and 30 minutes, respectively. This shows that formation of the dipeptide Asp-Phe really takes place on the dipeptide synthetase.

In a final experiment formation of Asp-Phe was also confirmed by comparing the HPLC retention times and mass-spectra of the dipeptide formed. In a total volume of 200 µl 50 pmol of Asp-Phe-TE-*His*₆ was incubated with lmM of ATP, 1 mM of Asp and 0.5 mM of Phe in a buffer (50 mM HEPES, 20 mM MgCl₂, pH 8) for 6 hours at 37 °C. To finish the reactions, 100 µl of n-butanol were added to precipitate the enzyme which then was removed. The remaining clear solution was evaporated *in vacuo* for 3 hours and replenished to a volume of 20 µl with 10 vol% of methanol in water.

For further analyses this volume was diluted 10x (again with 10 vol% methanol in water) and portions thereof then were subjected to HPL Cfollowed by photometric detection (as shown below) and to Electrospray Ionisation LC-MS analysis. Reference samples of Asp-Phe synthesised chemically were used for comparison.

For the HPLC with photometric detection a 50 µl portion of the 10x diluted sample was injected on a Chromsep Inertsil 5 ODS-3 column (250 x 3 mm; particle size 5 µ). Eluents A (0.05 M aq. H₃BO₃ buffer pH = 3.0) and B (acetonitrile; Merck, HPLC-grade) were used at a gradient (t = 0 min: 98% A, 2% B; t = 35 min: 10% A, 90% B) and a flow of 1.2 ml/min, at 40 °C.

Detection was done photometrically (at 210 nm and 257 nm, with quantification at 210 nm).

In the resulting HPLC chromatogram for the experimental sample a peak was found at 6.59 minutes which was at exactly the same retention time as for the Asp-Phe reference compound. Moreover, the UV-spectrum recorded for the Asp-Phe peak from the sample was shown to be identical (as to extinction versus wavelength in the region from 200 to 380 nm) to the one recorded for the Asp-Phe reference compound. The amount of Asp-Phe in the sample was calculated to be 16.1 mg/l.

For the Electrospray Ionisation LC-MS analysis a 4 µl portion of the lOx diluted experimental sample (see above) was injected onto the column. As a column a Nucleosil 120-3 C18 reversed-phase (Macherey & Nagel, 250 x 4 mm) was used. The eluents were eluents A (demi-water containing 0.05% of formic acid) and B (HPLC-grade methanol containing 0.05% of formic acid). As a gradient eluents A and B were used as follows: t = 0: 10%B; t = 25 min: 60% B; t = 30 min: 100% B; t = 34 min: 100% B at a flow of 0.4 ml/min at ambient temperature. Detection was done by total ion current (TIC) using electrospray ionisation in the positive ion mode as ionisation technique. The scan range was 120-300 amu with a dwell time of 1 msec. The retention time for Asp-Phe was 23.7 minutes (both for the sample and the reference compound). Mass spectroscopy confirmed the same molecular weight (280 g/mol) for both the Asp-Phe from the sample and the reference compound, and also identical fragmentation patterns were observed.
According to this technique the amount of Asp-Phe in the sample was calculated to be about 20 mg/l.

### Annex to the application documents-subsequently filed sequences listing

SEQUENCE LISTING

<110> Holland Sweetener Company V.O.F.

<120> Microbiological Production Method For
      Alpha-L-Aspartyl-L-Phenylalanine

<130> 4024ep

<140> 99203518.8
<141> 1999-10-27

<160> 47

<170> PatentIn Ver. 2.1

<210> 1
<211> 6
<212> PRT
<213> Artificial Sequence

<220>
<223> Description of Artificial Sequence:Consensus
      Sequence

<400> 1
Leu Thr Tyr Xaa Glu Leu
  1               5

<210> 2
<211> 6
<212> PRT
<213> Artificial Sequence

<220>
<223> Description of Artificial Sequence:Consensus
      Sequence

<400> 2
Leu Ser Tyr Xaa Glu Leu
  1               5

<210> 3
<211> 12
<212> PRT
<213> Artificial Sequence

<220>
<223> Description of Artificial Sequence:Consensus
      Sequence

<400> 3
Leu Lys Ala Gly Xaa Ala Tyr Leu Val Pro Leu Asp
 1               5                   10

<210> 4
<211> 12
<212> PRT
<213> Artificial Sequence

<220>
<223> Description of Artificial Sequence:Consensus
      Sequence

<400> 4
Leu Lys Ala Gly Xaa Ala Tyr Leu Leu Pro Leu Asp
 1               5                   10

<210> 5
<211> 12
<212> PRT
<213> Artificial Sequence

<220>
<223> Description of Artificial Sequence:Consensus
      Sequence

<400> 5
Leu Lys Ala Gly Xaa Ala Tyr Leu Val Pro Ile Asp
 1               5                   10

<210> 6
<211> 12
<212> PRT
<213> Artificial Sequence

<220>
<223> Description of Artificial Sequence:Consensus
      Sequence

<400> 6

Leu Lys Ala Gly Xaa Ala Tyr Leu Leu Pro Ile Asp
1               5                   10

<210> 7
<211> 16
<212> PRT
<213> Artificial Sequence

<220>
<223> Description of Artificial Sequence:Consensus
      Sequence

<400> 7
Leu Ala Tyr Xaa Xaa Tyr Thr Ser Gly Ser Thr Gly Xaa Pro Lys Gly
1               5                   10                  15

<210> 8
<211> 16
<212> PRT
<213> Artificial Sequence

<220>
<223> Description of Artificial Sequence:Consensus
      Sequence

<400> 8
Leu Ala Tyr Xaa Xaa Tyr Thr Ser Gly Thr Thr Gly Xaa Pro Lys Gly
1               5                   10                  15

<210> 9
<211> 4
<212> PRT
<213> Artificial Sequence

<220>
<223> Description of Artificial Sequence:Consensus
      Sequence

<400> 9
Phe Asp Xaa Ser
1

<210> 10
<211> 7

<212> PRT
<213> Artificial Sequence

<220>
<223> Description of Artificial Sequence:Consensus
      Sequence

<400> 10
Asn Xaa Tyr Gly Pro Thr Glu
  1               5

<210> 11
<211> 15
<212> PRT
<213> Artificial Sequence

<220>
<223> Description of Artificial Sequence:Consensus
      Sequence

<400> 11
Gly Glu Leu Xaa Ile Xaa Gly Xaa Gly Val Ala Arg Gly Tyr Leu
  1               5                  10                  15

<210> 12
<211> 15
<212> PRT
<213> Artificial Sequence

<220>
<223> Description of Artificial Sequence:Consensus
      Sequence

<400> 12
Gly Glu Leu Xaa Ile Xaa Gly Xaa Gly Leu Ala Arg Gly Tyr Leu
  1               5                  10                  15

<210> 13
<211> 6
<212> PRT
<213> Artificial Sequence

<220>
<223> Description of Artificial Sequence:Consensus
      Sequence

<400> 13
Tyr Arg Thr Gly Asp Leu
1               5

<210> 14
<211> 6
<212> PRT
<213> Artificial Sequence

<220>
<223> Description of Artificial Sequence:Consensus
      Sequence

<400> 14
Tyr Lys Thr Gly Asp Leu
1               5

<210> 15
<211> 20
<212> PRT
<213> Artificial Sequence

<220>
<223> Description of Artificial Sequence:Consensus
      Sequence

<400> 15
Gly Arg Xaa Asp Xaa Gln Val Lys Ile Arg Gly Xaa Arg Ile Glu Leu
1               5                   10                  15

Gly Glu Ile Glu
            20

<210> 16
<211> 7
<212> PRT
<213> Artificial Sequence

<220>
<223> Description of Artificial Sequence:Consensus
      Sequence

<400> 16
Leu Pro Xaa Tyr Met Ile Pro

1                 5


<210> 17
<211> 7
<212> PRT
<213> Artificial Sequence

<220>
<223> Description of Artificial Sequence:Consensus
      Sequence

<400> 17
Leu Pro Xaa Tyr Met Val Pro
  1               5


<210> 18
<211> 6
<212> PRT
<213> Artificial Sequence

<220>
<223> Description of Artificial Sequence:Consensus
      Sequence

<400> 18
Asn Gly Lys Val Asp Arg
  1               5


<210> 19
<211> 6
<212> PRT
<213> Artificial Sequence

<220>
<223> Description of Artificial Sequence:Consensus
      Sequence

<400> 19
Asn Gly Lys Leu Asp Arg
  1               5


<210> 20
<211> 12
<212> PRT

<213> Artificial Sequence

<220>
<223> Description of Artificial Sequence:Consensus
      Sequence

<400> 20
Asp Xaa Phe Phe Xaa Xaa Leu Gly Gly His Ser Leu
  1               5                   10

<210> 21
<211> 12
<212> PRT
<213> Artificial Sequence

<220>
<223> Description of Artificial Sequence:Consensus
      Sequence

<400> 21
Asp Xaa Phe Phe Xaa Xaa Leu Gly Gly Asp Ser Leu
  1               5                   10

<210> 22
<211> 12
<212> PRT
<213> Artificial Sequence

<220>
<223> Description of Artificial Sequence:Consensus
      Sequence

<400> 22
Asp Xaa Phe Phe Xaa Xaa Leu Gly Gly His Ser Ile
  1               5                   10

<210> 23
<211> 12
<212> PRT
<213> Artificial Sequence

<220>
<223> Description of Artificial Sequence:Consensus
      Sequence

<400> 23
Asp Xaa Phe Phe Xaa Xaa Leu Gly Gly Asp Ser Ile
1               5                   10

<210> 24
<211> 10
<212> PRT
<213> Artificial Sequence

<220>
<223> Description of Artificial Sequence:Consensus
      Sequence

<400> 24
Ser Xaa Ala Gln Xaa Arg Leu Trp Xaa Leu
1               5                   10

<210> 25
<211> 10
<212> PRT
<213> Artificial Sequence

<220>
<223> Description of Artificial Sequence:Consensus
      Sequence

<400> 25
Ser Xaa Ala Gln Xaa Arg Met Trp Xaa Leu
1               5                   10

<210> 26
<211> 10
<212> PRT
<213> Artificial Sequence

<220>
<223> Description of Artificial Sequence:Consensus
      Sequence

<400> 26
Ser Xaa Ala Gln Xaa Arg Leu Tyr Xaa Leu
1               5                   10

<210> 27
<211> 10
<212> PRT
<213> Artificial Sequence

<220>
<223> Description of Artificial Sequence:Consensus
      Sequence

<400> 27
Ser Xaa Ala Gln Xaa Arg Met Tyr Xaa Leu
 1               5                   10



<210> 28
<211> 9
<212> PRT
<213> Artificial Sequence

<220>
<223> Description of Artificial Sequence:Consensus
      Sequence

<400> 28
Arg His Glu Xaa Leu Arg Thr Xaa Phe
 1               5



<210> 29
<211>' 10
<212> PRT
<213> Artificial Sequence

<220>
<223> Description of Artificial Sequence:Consensus
      Sequence

<400> 29
Met His His Xaa Ile Ser Asp Gly Trp Ser
 1               5                   10



<210> 30
<211> 10
<212> PRT
<213> Artificial Sequence

<220>

<223> Description of Artificial Sequence:Consensus
      Sequence

<400> 30
Met His His Xaa Ile Ser Asp Gly Val Ser
  1               5                  10

<210> 31
<211> 7
<212> PRT
<213> Artificial Sequence

<220>
<223> Description of Artificial Sequence:Consensus
      Sequence

<400> 31
Tyr Xaa Asp Phe Ala Val Trp
  1               5

<210> 32
<211> 7
<212> PRT
<213> Artificial Sequence

<220>
<223> Description of Artificial Sequence:Consensus
      Sequence

<400> 32
Tyr Xaa Asp Tyr Ala Val Trp
  1               5

<210> 33
<211> 11
<212> PRT
<213> Artificial Sequence

<220>
<223> Description of Artificial Sequence:Consensus
      Sequence

<400> 33
Ile Gly Xaa Phe Val Asn Thr Gln Cys Xaa Arg
  1               5                  10

<210> 34
<211> 11
<212> PRT
<213> Artificial Sequence

<220>
<223> Description of Artificial Sequence:Consensus
      Sequence

<400> 34
Val Gly Xaa Phe Val Asn Thr Gln Cys Xaa Arg
  1               5                   10


<210> 35
<211> 11
<212> PRT
<213> Artificial Sequence

<220>
<223> Description of Artificial Sequence:Consensus
      Sequence

<400> 35
Ile Gly Xaa Phe Val Asn Thr Leu Cys Xaa Arg
  1               5                   10


<210> 36
<211> 11
<212> PRT
<213> Artificial Sequence

<220>
<223> Description of Artificial Sequence:Consensus
      Sequence

<400> 36
Val Gly Xaa Phe Val Asn Thr Leu Cys Xaa Arg
  1               5                   10


<210> 37
<211> 11
<212> PRT
<213> Artificial Sequence

<220>
<223> Description of Artificial Sequence:Consensus
      Sequence

<400> 37
Ile Gly Xaa Phe Val Asn Thr Gln Ala Xaa Arg
  1               5                   10


<210> 38
<211> 11
<212> PRT
<213> Artificial Sequence

<220>
<223> Description of Artificial Sequence:Consensus
      Sequence

<400> 38
Val Gly Xaa Phe Val Asn Thr Gln Ala Xaa Arg
  1               5                   10


<210> 39
<211> 11
<212> PRT
<213> Artificial Sequence

<220>
<223> Description of Artificial Sequence:Consensus
      Sequence

<400> 39
Ile Gly Xaa Phe Val Asn Thr Leu Ala Xaa Arg
  1               5                   10


<210> 40
<211> 11
<212> PRT
<213> Artificial Sequence

<220>
<223> Description of Artificial Sequence:Consensus
      Sequence

<400> 40

Val Gly Xaa Phe Val Asn Thr Leu Ala Xaa Arg
  1               5                   10

<210> 41
<211> 7
<212> PRT
<213> Artificial Sequence

<220>
<223> Description of Artificial Sequence:Consensus
      Sequence

<400> 41
His Gln Asp Tyr Pro Phe Glu
  1               5

<210> 42
<211> 7
<212> PRT
<213> Artificial Sequence

<220>
<223> Description of Artificial Sequence:Consensus
      Sequence

<400> 42
Asn Gln Asp Tyr Pro Phe Glu
  1               5

<210> 43
<211> 7
<212> PRT
<213> Artificial Sequence

<220>
<223> Description of Artificial Sequence:Consensus
      Sequence

<400> 43
His Gln Asp Val Pro Phe Glu
  1               5

<210> 44

<211> 7
<212> PRT
<213> Artificial Sequence

<220>
<223> Description of Artificial Sequence:Consensus
      Sequence

<400> 44
Asn Gln Asp Val Pro Phe Glu
  1               5


<210> 45
<211> 8
<212> PRT
<213> Artificial Sequence

<220>
<223> Description of Artificial Sequence:Consensus
      Sequence

<400> 45
Arg Asp Xaa Ser Arg Asn Pro Leu
  1               5


<210> 46
<211> 5
<212> PRT
<213> Artificial Sequence

<220>
<223> Description of Artificial Sequence:Consensus
      Sequence

<400> 46
Gly His Ser Xaa Gly
  1               5


<210> 47
<211> 5
<212> PRT
<213> Artificial Sequence

<220>
<223> Description of Artificial Sequence:Consensus

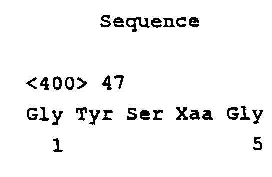

## Claims

1. Method for the microbiological production of α-L-aspartyl-L-phenylalanine (Asp-Phe) from the substrates L-aspartic acid (L-Asp) and L-phenylalanine (L-Phe) characterised in that the substrates are contacted, in the presence of an effective amount of adenosine-triphosphate (ATP), with a non-ribosomal dipeptide synthetase comprising two minimal modules connected by one condensation domain wherein the N-terminal module of these modules is recognising L-aspartic acid and the C-terminal module of these modules is recognising L-phenylalanine and is covalently bound at its N-terminal end to the condensation domain, and wherein each of these minimal modules is composed of an adenylation domain and a 4'-phosphopantetheinyl cofactor containing thiolation domain, and that the α-L-aspartyl-L-phenylalanine (Asp-Phe) formed is recovered.

2. Method for the production of Asp-Phe according to claim 1, characterised in that the condensation domain in the dipeptide synthetase is connected to both minimal modules in such way that it is also covalently bound to the module recognising L-aspartic acid.

3. Method for the production of Asp-Phe according to claim 1 or 2, characterised in that also a releasing factor is present for the Asp-Phe formed on the dipeptide synthetase.

4. Method for the production of Asp-Phe according to any of claims 1 to 3, characterised in that the releasing factor is a protein which shows thioesterase-like functions and forms an integrated domain of the dipeptide synthetase at the C-terminus thereof.

5. Method for the production of Asp-Phe according to any of claims 1 to 4, characterised in that prior to the production of Asp-Phe the dipeptide synthetase has undergone optimisation for its function by using one or more post-translational modifying activities.

6. Method for the production of Asp-Phe according to claim 5, characterised in that as an post-translational modifying activity a 4'-phosphopantetheinyl (4'-PP) transferase is used.

7. Method for the production of Asp-Phe according to any of claims 1 to 6, characterised in that also a non-integrated protein with thioesterase Type-II-like activity is present together with the dipeptide synthetase.

8. Method for the production of Asp-Phe according to any of claims 1 to 7, characterised in that the dipeptide synthetase is present in living cell-material of a micro-organism, and that glucose, L-Asp and/or L-Phe are being fed to said fermentor, and that the Asp-Phe formed is recovered.

9. Method for the production of Asp-Phe according to claim 8 characterised in that the micro-organism is first grown in a fermentor to reach a predetermined cell density before the expression of the Asp-Phe dipeptide synthetase is switched on and feeding of the glucose, L-Asp and/or L-Phe for the synthesis of the Asp-Phe dipeptide is started.

10. Method for the production of Asp-Phe according to claim 9, characterised in that the micro-organism is an L-phenylalanine producing micro-organism and that only glucose and L-Asp are being fed.

11. Method for the production of Asp-Phe according to claim 10, characterised in that the micro-organism is an *Escherichia* or *Bacillus species*.

12. Method for the production of Asp-Phe according to any of claims 8 to 11, characterised in that the micro-organism used is a strain with reduced protease activity for Asp-Phe or lacking such activity towards Asp-Phe.

13. Method for the production of Asp-Phe according to any of claims 8 to 12, characterised in that the micro-organism used also contains a suitable export system for Asp-Phe formed and/or one or more suitable up-take system(s) for glucose and/or L-Asp and/or L-Phe.

14. Method for the production of Asp-Phe according to any of claims 1 to 7, characterised in that the production of Asp-Phe is carried out *in vitro* in an enzyme reactor, while ATP is supplied, and L-Asp and/or L-Phe are being fed, and the Asp-Phe formed is recovered.

15. Method for the production of Asp-Phe according to claim 14, characterised in that the supply of ATP is provided in part by an in situ ATP-regenerating system.

16. Method for the production of Asp-Phe according to claim 15, characterised in that the ATP-regenerating system is present in a permeabilised micro-organism.

17. A DNA fragment or a combination of DNA fragments coding for a non-ribosomal Asp-Phe dipeptide synthetase, which synthetase comprises two minimal modules connected by one condensation domain wherein the N-terminal module of these modules is recognising L-aspartic acid and the C-terminal module of these modules is recognising L-phenylalanine and is covalently bound at its N-terminal end to the condensation domain, and wherein each of these minimal modules is composed of an adenylation domain and a 4'-phosphopantetheinyl cofactor containing thiolation domain.

18. A DNA fragment coding for an Asp-Phe dipeptide synthetase according to claim 17, characterised in that the condensation domain in the encoded dipeptide synthetase is connected to both minimal modules in such way that it is also covalently bound to the module recognising L-aspartic acid.

19. A DNA fragment according to claim 17 or 18, or a combination of DNA fragments according to claim 17, characterised in that the DNA fragment or combination of DNA fragments encoding the dipeptide synthetase also code for a releasing factor for the Asp-Phe formed on that dipeptide synthetase.

20. A DNA fragment or a combination of DNA fragments according to any of claims 17 to 19, characterised in that it/they also code for a protein which shows thioesterase-like functions and forms an integrated domain of the dipeptide synthetase at the C-terminus thereof.

21. A DNA fragment or a combination of DNA fragments according to any of claims 17 to 20, characterised in that it/they also express(es) a post-translational modifying activity for efficient non-ribosomal synthesis of Asp-Phe on the synthetase.

22. A DNA fragment or a combination of DNA fragments according to claim 21, characterised in that the post-translational modifying activity expressed is a 4'-phosphopantetheinyl (4'-PP) transferase activity.

23. A DNA fragment or a combination of DNA fragments according to any of claims 17 to 22, characterised in that it/they also code for a non-integrated protein with thioesterase Type-II-like activity.

24. A micro-organism containing a DNA fragment or a combination of DNA fragments according to any of claims 17-23.

25. A micro-organism according to claim 24 wherein the micro-organism is capable of producing L-Asp and/or L-Phe.

26. A micro-organism according to claim 25 wherein the micro-organism is an *Escherichia coli* or *Bacillus species.*

27. Asp-Phe dipeptide synthetase characterised in that it comprises two minimal modules connected by one condensation domain wherein the N-terminal module of these modules is recognising L-aspartic acid and the C-terminal module of these modules is recognising L-phenylalanine and is covalently bound at its N-terminal end to the condensation domain, and wherein each of these minimal modules is composed of an adenylation domain and a 4'-phosphopantetheinyl cofactor containing thiolation domain.

28. Asp-Phe dipeptide synthetase according to claim 27 characterised in that the condensation domain in the dipeptide synthetase is connected to both minimal modules in such way that it is also covalently bound to the module recognising L-aspartic acid.

29. Asp-Phe dipeptide synthetase according to claim 27 or 28, characterised in that the dipeptide synthetase also comprises a releasing factor for the Asp-Phe formed on that dipeptide synthetase.

30. Asp-Phe dipeptide synthetase according to claim 29, characterised in that the releasing factor is a protein which shows thioesterase-like functions and forms an integrated domain of the dipeptide synthetase at its C-terminus.
